# EUROPEAN PATENT SPECIFICATION

(11) **EP 3 852 702 B1**
(45) Date of publication and mention of the grant of the patent: **06.07.2022**
(21) Application number: 19769792.3
(22) Date of filing: 16.09.2019
(51) Int. Cl.: A61F 9/008

(54) **APPARATUS FOR CONTROLLED PLUME EVACUATION**
VORRICHTUNG ZUR KONTROLLIERTEN RAUCHGASABSAUGUNG
APPAREIL D'ÉVACUATION CONTRÔLÉE DE PANACHE

(30) Priority: 20.09.2018 DE 102018216088
(43) Date of publication of application: 28.07.2021
(73) Proprietor: IVIS TECHNOLOGIES S.r.l, 74100 Taranto (IT)
(72) Inventor: D' IPPOLITO, Giuseppe, 74100 Taranto (IT)
(74) Representative: Bardehle Pagenberg Partnerschaft mbB Patentanwälte Rechtsanwälte
(86) International application number: PCT/EP2019/074717
(87) International publication number: WO 2020/058198

(56) References cited:
- EP-A1- 0 224 322
- US-A- 5 741 245
- US-A1- 2015 025 444

## Description

### 1. Technical Field:

The present invention generally relates to laser systems for corneal tissue ablation, and in particular to an apparatus for providing a controlled atmosphere in a region between a laser system and a cornea.

### 2. Description of the prior art:

Lasers, in particular excimer lasers, are widely used to cure visual impairments due to refractive errors. The energy delivered onto the cornea by the laser removes corneal tissue through a photo-ablative process, hence correcting the cornea's shape and thereby its refractive error.

For the sake of accuracy, lasers with a small beam diameter, or spot size, e.g., below 1 mm, are typically used. This in turn renders such lasers or their accuracy, respectively, highly dependent on environmental conditions. Environmental parameters such as temperature, air pressure or humidity influence the amount of energy delivered in a laser beam emitted by the laser. A laser beam with a small diameter may also be partly absorbed or otherwise impaired by, e.g., particles along its path.

In fact, a significant amount of such interfering particles emerges from the corneal surface as a consequence of the operation of the laser itself as, due to the photo-ablative process, it causes local evaporations of the corneal tissue known as "plumes". In addition to affecting the laser accuracy, plumes are also carcinogenic, such that there is a twofold reason to evacuate them quickly.

There are laser systems known in the state of the art that do so using fans or aspirators located at predefined distances from the eye receiving surgery.

However, even when using the fans or aspirators known in the state of the art, the laser ablation may not always be sufficiently predictable, particularly when it comes to high precision surgery. Therefore, there is a need to improve the predictability of corneal tissue removal by laser systems to further improve the precision of refractive eye surgery.

Devices which are attached to the eyeball are known from US 2013/0035672 A1, US 5,425,727 A, US 5,360,424 A, and DE 36 12 287 A1. However, such attachment to the eyeball is extremely inconvenient for the patient.

The eye piece taught by EP 0 224 322 A1 does not comprise a sleeve adapted to provide a fit with the face, as set out in present claim 1.

### 3. Summary of the invention:

The above need is at least partly met by an apparatus according to independent claim 1. In an example, an apparatus for providing a controlled atmosphere in a region between a laser system for corneal tissue ablation and an eye of a patient comprises means for isolating the region between the laser system and the eye and means for controlling the atmosphere in the region.

Thus, an apparatus may be provided that allows to control and keep steady the environmental conditions in the region a laser beam traverses when aimed at the eye of the patient. In particular, the region may be isolated from unpredictable disturbances which not even the most modern and sophisticated treatment rooms may avoid, e.g., such caused by coughing of the patient or movements of medical personnel. This increases the accuracy of the laser and hence the reliability and safety of the corresponding treatment.

Moreover, the atmospheric conditions within the region may thus be controlled independently from those of the treatment room. This may allow to optimize the conditions in the region between the laser system and the eye with respect to the accuracy of the laser, while the overall conditions in the treatment room may be adjusted according to the requirements and preferences of the medical personnel. In fact, the invention may even allow employing a laser system for corneal tissue ablation outside of specialized and sophisticated treatment rooms, increasing the flexibility and accessibility of corresponding treatments.

The means for isolating may circumscribe the region between the laser system and the eye at least partly. In some examples, the means for isolating may also circumscribe the region fully, hence providing particularly good isolation. For example, the means for isolating may circumscribe an optical axis of the laser system, e.g., in an essentially radially symmetric manner, such that the means for isolating comprise a truncated conical or cylindrical shape.

At least a portion of the means for isolating the region between the laser system and the eye may be extensible. This may allow adjustment of the apparatus with respect to a position of the patient's eye. In some examples, the apparatus may then comprise at least an extended and a retracted position. Particularly, this may facilitate patient centration below the laser while such apparatus is in the retracted position.

The apparatus may further comprise means for extending the extensible portion along an optical axis of the laser system. For example, such means may be located remotely from the means for isolating. This may allow medical personnel to adjust the extension without immediately reaching for the means for isolating. Moreover, any adjustment may then be initiated remotely from the laser system and the patient, in a convenient position and posture. Such means for extending may also allow for automatic adjustment of the extension as they may, for example, be coupled to the laser system employing the apparatus, but also, e.g., to suitable sensors. Additionally, means for extending the extensible portion may also function to fixate the degree of extension as well as the position of the means for isolating such that accidental changes may be avoided.

The means for isolating the region between the laser system and the eye comprises an eye piece adapted to be arranged around the eye of the patient, e.g., in a flush manner. This may ensure that there is no gap between a periphery of the eye of the patient that is to be in direct contact with the eye piece and the means for isolating such that the region between the laser system and the eye is effectively isolated. In some examples, the eye piece may be adapted to the general human physiognomy or to the patient's physiognomy in particular. The eye piece may also provide cushioning. All of this may increase the comfort of the patient, already for the reason that there is no need to press, e.g., the eye socket overly strongly against the means for isolating to ensure proper isolation.

The eye piece may be removable. This may allow for easy and repeated sterilization and hence reuse of the eye piece. In another example, this may allow the use of disposable eye pieces. Also, this may allow to use eye pieces of different sizes and/or geometry with the apparatus according to the physiognomy of the individual patient. The eye piece may even be individually adjusted or manufactured.

The means for isolating may be adapted to be arranged around a laser outlet of the laser system in a flush manner. This may avoid any gaps between the means for isolating and the laser system, preventing particles from entering the region between the laser system and the eye close to the laser system such that the region is effectively isolated. This may be desirable as particles entering the laser's path close to the laser outlet may have the most significant impact in terms of deflection.

The means for controlling the atmosphere may be adapted to ventilate at least a portion of the region between the laser system and the eye. This may allow plumes to be removed, e.g., by diffusion, out of the laser's path more effectively. Particularly, a portion of the region located immediately above/in front of the eye undergoing treatment may be ventilated to quickly remove plumes right where they emerge.

The means for controlling the atmosphere may be adapted to ventilate the portion of the region by providing an airflow through the region. An airflow may actively remove plumes from the laser's path and hence accelerate plume evacuation, in turn increasing the efficiency of the treatment. In some examples, the air may flow essentially perpendicular to the optical axis of the laser system in order to quickly remove plumes from the laser's path. In other examples, the air may also flow at least in parts along the direction of the optical axis in order to ensure that carcinogenic plumes are guided away from the patient.

The airflow may be provided from at least one air inlet to at least one air outlet of the means for isolating. This may allow to remove plumes not only from the laser's path and/or from the periphery of the eye, but from the region between the laser system and the eye of the patient altogether. This may avoid the region from saturating with plume particles and may also increase the safety of the treatment given the carcinogenic properties of plumes. In some examples, the region between the laser system and the eye of the patient may be essentially sealed from the environment. Apart from the one or more air inlets and air outlets, which allow a controlled air flow, no gaps or openings may be present in the means for isolating.

The at least one air inlet and/or the at least one air outlet may be provided in the eye piece. This in turn provides an airflow immediately above/in front of the eye undergoing treatment, allowing plumes to be removed right where they emerge. Moreover, while an air inlet may be provided in the eye piece, an air outlet in the means for isolating may be provided further away from the eye such that the airflow is directed away from the eye of the patient. This increases efficiency and safety of the treatment further.

More generally, and irrespective of whether the means for isolating comprise an eye piece, one or more air inlets in the means for isolating may be arranged closer to the eye undergoing treatment than one or more (or all) air outlets in the means for isolating in order to ensure that carcinogenic plumes are guided away from the patient and his or her eye.

The means for controlling the atmosphere may further comprise a vacuum pump which is connected to the at least one air outlet. Each air outlet may be connected to one or more vacuum pumps. This may allow to control and/or adjust, e.g., to increase, the airflow and hence speed up plume evacuation further, as needed, increasing efficiency and safety of the corresponding treatment. Also, the use of a pump may ensure a constant airflow such that overall environmental conditions in the region between the laser system and the eye of the patient stay steady and plumes are evacuated homogenously. This may in turn improve the accuracy and stability of the laser as well as the reliability and predictability of the corresponding treatment. It is noted that by controlling the pump, the flow of air through the one or more air outlets may be controlled as well as the flow of air through the one or more air inlets.

The means for controlling the atmosphere may further comprise a filter. This may prevent plume particles from spreading, e.g., throughout the treatment room. This is highly desirable due to their carcinogenic nature, in turn increasing the safety of the treatment. In some examples, such a filter may be arranged at one or more air outlets in the means for isolating. In other examples, such a filter may be arranged between an air outlet and an intake of a pump comprised in the means for controlling the atmosphere. In yet other examples, a filter may be arranged at an outlet of such a pump.

The apparatus described above may further comprise means for illuminating the eye. This may allow for better targeting of the laser. For example, the means for illuminating may comprise visible light. This may help manual targeting of the laser as performed by a surgeon. In another example, the means for illuminating may comprise infrared light. This may enable sensor-based laser targeting, e.g., based on an eye tracker. Moreover, illumination of the eye by infrared light may enable the use of infrared-based proximity sensors that may help to guide and/or control the means for extending. In yet other examples, sensors based on a different frequency spectrum may be used and the means for illumination may be adapted accordingly. Apart from the described sensors also sensors of a different technology may be used to provide proximity sensor functionality such as to guide and/or control the means for extending.

According to another aspect, the present invention is directed to a laser system for corneal tissue ablation comprising an apparatus as described herein. Such a laser system may allow for more accurate and predictable laser operation and hence for increasingly reliable and safe performance of refractive eye surgery as described above. Moreover, this may enable an integrated control of both the laser system and the apparatus by common control units and/or power supplies. For example, an extension of the apparatus may be coupled to operation of the laser, such that the laser may only emit radiation when the means for isolation are extended. However, this is not mandatory, and the apparatus may also be provided as an independent unit which is merely attached or otherwise connected to the laser system.

### 4. Brief description of the Figures:

Possible examples of the present invention will be described in more detail in the subsequent detailed description with reference to the following figures:
Figs. 1A, 1B: Example of an apparatus according to the present invention;
Fig. 2A-2C: Example of an extensible portion of an apparatus according to the present invention;
Fig. 3A-3D: Example of an eye piece of an apparatus according to the present invention;
Fig. 4A-4D: Example of means for controlling an atmosphere according to the present invention;
Figs. 5A-5D: Example of an apparatus comprising means for extending according to the present invention;
Fig. 6A, 6B: Example of a laser system comprising an apparatus according to the present invention.

### 5. Detailed description of possible examples:

For the sake of brevity only a few examples will be described in the following. The skilled person will recognize that the specific features described with reference to these examples may be modified and combined differently and that individual features may also be omitted if they are not essential. The general explanations in the sections above will also be valid for the following more detailed explanations.

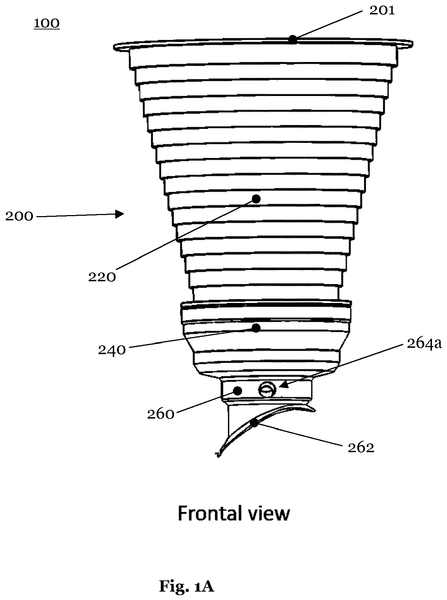

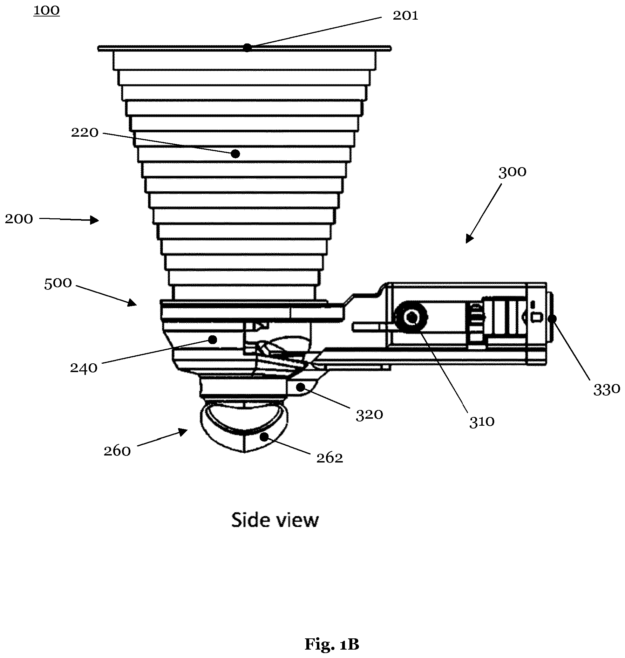

Figs. 1A, 1B show front and side views of an apparatus 100 for providing a controlled atmosphere in a region between a laser system for corneal tissue ablation and an eye of a patient. Apparatus 100 comprises means for isolating 200 the region between the laser system and the eye and means for controlling 300 the atmosphere in the region. Means for isolating 200 comprise extensible portion 220, intermediate portion 240 as well as eye piece 260. The various portions such as portions 220, 240 and eye piece 260 may be attached to each other using one or more threads, but they may also be connected by other means, e.g., they may be clipped and/or glued together.

Means for isolating 200 are adapted to circumscribe the region between the laser system for corneal tissue ablation (which is to be located at an upper end of means for isolating 200 as shown in Fig. 1A) and an eye (which is to be located below eye piece 260 as shown in Fig. 1A). In the example of Figs. 1A, 1B, means for isolating 200 comprise an essentially truncated conical shape, and correspondingly a region of an essentially truncated conical shape is circumscribed. In other examples, means for isolating 200 may circumscribe a region of an essentially cylindrical shape. More generally, means for isolating 200 may circumscribe an optical axis of the laser system (which is arranged vertically in the example of Fig. 1A) in a symmetric manner, e.g., such that they comprise a radially symmetric shape with respect to the optical axis. In other examples, means for isolating 200 may only comprise partly symmetric shapes, or none at all.

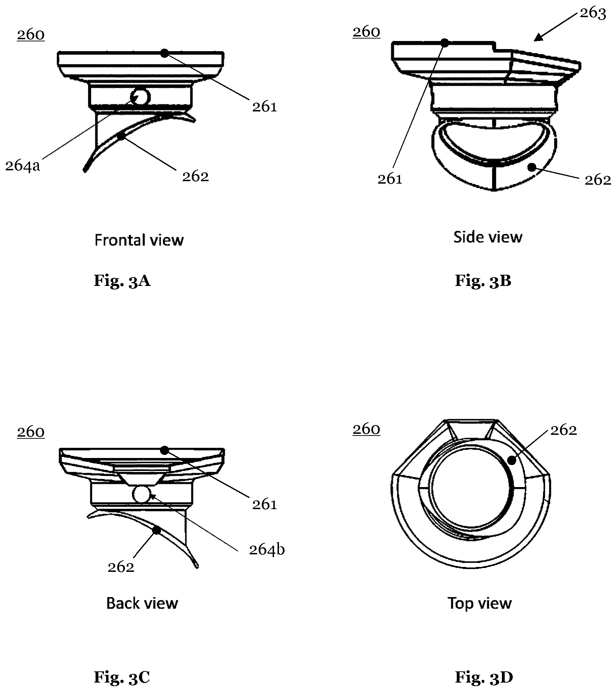

In the example of Figs. 1A, 1B, means for isolating 200 circumscribe the region between the laser system and the eye of the patient fully, except for an air inlet 264a in eye piece 260, such that a full sealing of the region is provided. See Figs. 3A-3D and below for details regarding eye piece 260. More generally, means for isolating 200 may be provided which fully circumscribe the region, possibly except for one or more air inlets and/or air outlets such as air inlet 264a and air outlet 264b, wherein the airflow through the air inlets and/or air outlets may then however be controlled. In other examples, means for isolating may circumscribe the region only partly.

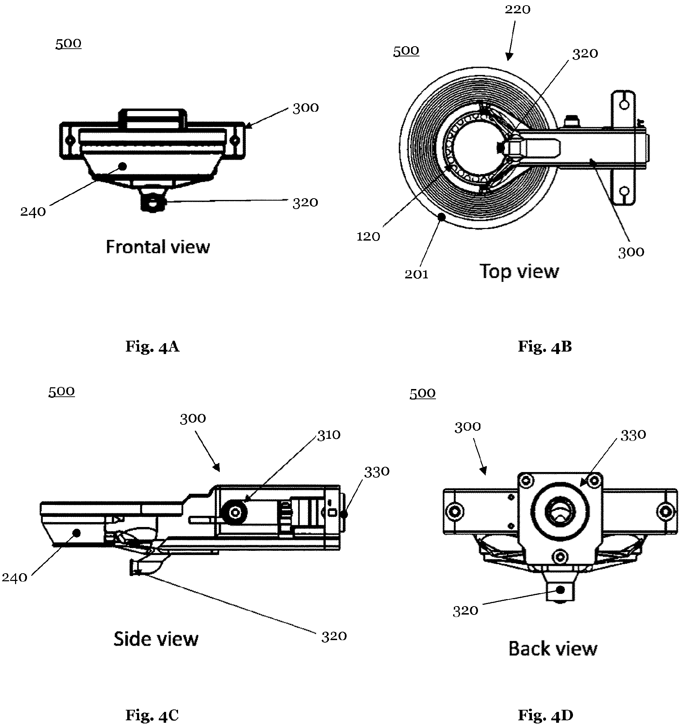

As shown in Fig. 1B, means for controlling 300 the atmosphere comprise a pump 310, an intake 320 and an outlet 330. In the example, means for controlling 300 and intermediate portion 240 of means for isolating 200 form an integral unit 500. See Figs. 4A-4D and below for details regarding unit 500 and means for controlling 300. It is noted that the integration of means for controlling 300 into integral unit 500 is only exemplary. In some examples, means for controlling 300 may be provided independently, e.g., for attachment to extensible portion 220, eye piece 260 or any other portion of means for isolating 200, or they may be an integral part of any portion of means for isolating 200. Also, in some examples, an intermediate portion 240 may not be provided.

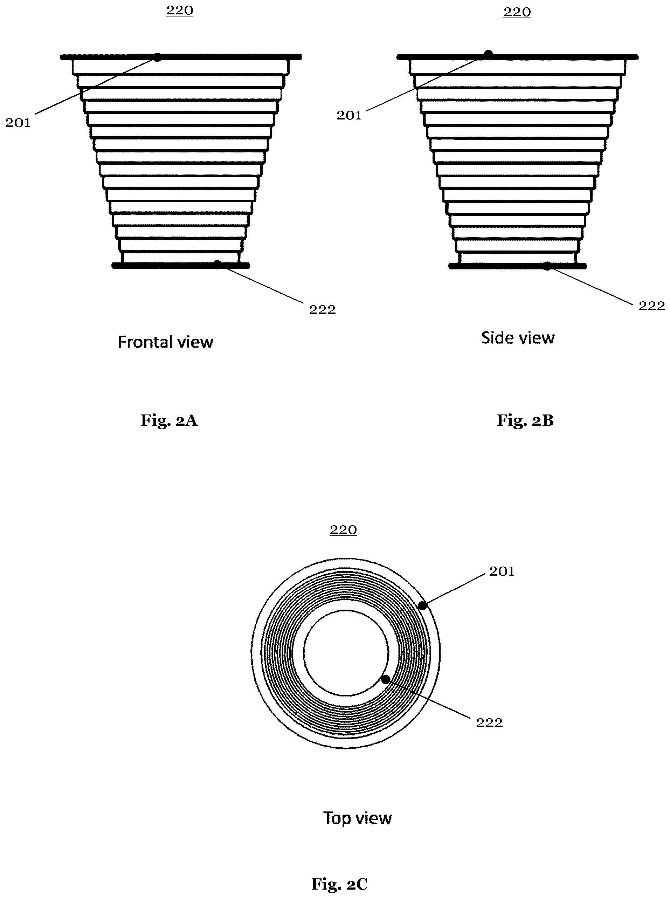

Figs. 2A-2C show different views of extensible portion 220 of means for isolating 200. Extensible portion 220 is radially symmetric and circumscribes an essentially truncated conical shape. This may benefit diffusion of plumes into a wider portion and away from the optical axis. As described above, other shapes are also possible. In other examples, means for isolating 200 may comprise a portion with the features described herein with regard to extensible portion 220, but without being extensible. For example, such a portion may be provided in the shape of extensible portion 220 in an extended position (as shown in Fig. 2A).

Extensible portion 220 may be extended and collapsed by moving rims 201 and 222 towards or away from each other. In the example, extensible portion 220 comprises a plurality of isolating elements that may slide into each other, i.e., extensible portion 220 may be extended and collapsed like a telescope. In other examples, a different mechanism may be used. For example, extensible portion 220 may comprise a textile connecting rims 201 and 222 that may be extended and collapsed. Such a textile may comprise an elastic and/or a non-elastic material. In yet other examples, extensible portion 220 may fold, e.g., onto itself or away from and/or towards the optical axis of the laser system.

Rim 201 forms the upper end of extensible portion 220 as well as of means for isolating 200. Rim 201 may optionally be adapted to be arranged around a laser outlet of the laser system in a flush manner. In the example of Figs. 2A-2C, rim 201 is adapted to be flush with a planar surface of the laser system, particularly such that a contact area between rim 201 and the laser system may be arranged around the laser outlet of the laser system. For example, the contact area may comprise an essentially circular shape. However, rim 201 may be provided in any other, e.g., non-circular, shape according to the shape of the periphery of the laser outlet such that there are no gaps between the laser system and rim 201 and means for isolating 200, respectively.

Rim 222 forms a lower end of extensible portion 220 and, in the present example, is adapted to receive intermediate portion 240 of unit 500. In other examples, rim 222 may be adapted to receive eye piece 260. In yet other examples, rim 222 may be adapted to be positioned around the eye of the patient directly. Both rims 201 and 222 may comprise one or more threads or any other means for attachment as described above.

Figs. 3A-3D show different views of eye piece 260. Eye piece 260 comprises an attachment portion 261 adapted to be connected to intermediate portion 240 of unit 500. In other examples, attachment portion 261 may (additionally) be adapted to be attached to any other portion of means for isolating 200, e.g., by a thread or any other means discussed above. For example, attachment portion 261 may be adapted to be connected to extensible portion 220, e.g., to its rim 222, and/or to the laser system. In some examples, eye piece 260 may be attached releasably. This may allow easy sterilization and hence reuse of the eye piece, but also its complete removal and disposal. In other examples, eye piece 260 may be disposable by design. For example, a centration procedure with respect to the patient's eye may be carried out while no eye piece is attached to the apparatus, e.g., using means for illuminating of apparatus 100 (e.g., as further described below), and an eye piece may be attached to apparatus 100 upon centration. Subsequently, extensible portion 220 may be brought into an extended position, such as to position the eye piece around the patient's eye.

Eye piece 260 also comprises a sleeve 262 adapted to be arranged around the eye undergoing treatment. Sleeve 262 is adapted to provide a (preferably tight) fit with the face , i.e., any gaps between eye piece 260 and the patient are avoided. For example, eye piece 260 and/or sleeve 262 may be adjustable. However, a high degree of flexibility and adaptability in this regard may also be provided if eye piece 260 and/or sleeve 262 is attached releasably. This way, eye pieces such as eye piece 260 and/or sleeves such as sleeve 262 comprising different shapes and sizes may be attached as needed. In some examples, eye piece 260 and/or sleeve 262 may be specifically manufactured according to the individual patient's physiognomy.

In the present example, eye piece 260 comprises an air inlet 264a and an air outlet 264b. In other examples, eye piece 260 may comprise a plurality of air inlets and/or air outlets such as air inlet 264a and air outlet 264b. In yet other examples, eye piece 260 may only comprise one or more air inlets such as air inlet 264a. Alternatively, eye piece 260 may only comprise one or more air outlets such as air outlet 264b. Possibly, eye outlet 260 may not comprise any air inlets or outlets. Rather, air inlets and air outlets such as air inlet 264a and air outlet 264b - if any - may be located in different portions of means for isolating 200. Particularly, one or more air outlets such as air outlet 264b may be positioned further away from the eye undergoing treatment than one or more air inlets such as air inlet 264a such that an airflow between the one or more air inlets and the one or more air outlets is directed away from the patient. For example, one or more air inlets may be provided in eye piece 260 such as air inlet 264a, whereas one or more air outlets may be provided in extensible portion 220 and/or intermediate portion 240. This may avoid contact between the patient and carcinogenic plumes. Attachment portion 261 comprises a recess 263 as shown in Fig. 3B (also visible in Figs. 3C, 3D). Recess 263 is adapted to receive a portion of unit 500, particularly a periphery of intake 320 as shown in Fig. 1B. In the example, recess 263 and air outlet 264b of eye piece 260 are arranged such that intake 320 of means for controlling 300 and unit 500, respectively, is flush with, i.e., tightly connects to, air outlet 264b.

Figs. 4A-4D show unit 500 which comprises means for controlling 300 the atmosphere in the region between the laser system and the eye of the patient and an optional intermediate portion 240. By means of intermediate portion 240, which is adapted to be arranged between extensible portion 220 and eye piece 260, means for controlling 300 may be connected to means for isolating 200. Intermediate portion 240 may comprise an approximately circular shape. Generally, the shape of intermediate portion 240 may be adapted to be connected to extensible portion 220 and/or eye piece 260. Specifically, in the example of Figs. 4A-4D, means for controlling 300 may thus be arranged adjacent to means for isolating 200. However, in other examples, means for controlling 300 may not form part of an integral unit such as unit 500 and may therefore be arranged in a different position, e.g., remote from means for isolating 300, but may be directly attached to means for isolating 300, as well.

Means for controlling 300 further comprise a vacuum pump 310, aspirating air through intake 320 and emitting it through outlet 330. In other examples, these elements may function the other way around, i.e., vacuum pump 310 may function as a pressure pump aspirating air, such that the air inlets and air outlets of means for isolating 200 as well as intake 320 and outlet 330 of vacuum pump 310 would reverse their functions. In yet other examples, means for controlling 300 may not comprise a pump at all. Rather, means for isolating 200 may be passively ventilated, e.g., by an air flow from an air inlet such as air inlet 264a of eye piece 260 to an air outlet such as air outlet 264b of eye piece 260. In some examples, means for controlling 300 may not use any air inlets and/or outlets such as air inlet 264a and air outlet 264b of eye piece 260. Rather, air may be circulated within means for isolating 200. Particularly, an airflow may be provided that guides particles such as plume particles away from the optical axis of the laser system and hence out of the laser's path, and/or away from the patient's eye, e.g., into the wider portions of essentially conically shaped means for isolating 200.

Means for controlling 300 the atmosphere may furthermore comprise a filter. In particular, such a filter may be arranged wherever air is ejected from apparatus 100. This may be at outlet 330 of means for controlling 300, but also at one or more air outlets such as air outlet 264b. Such a filter may also be comprised in pump 310, or it may be arranged upstream and/or downstream from pump 310.

Apparatus 100 may further comprise means for illuminating 120 the eye undergoing treatment. In the present example, means for illuminating 120 are arranged on unit 500, in particular on intermediate portion 240, as shown in Fig. 4B. Means for illuminating may comprise a circular shape and may be arranged concentrically with the optical axis of the laser system such that a laser beam emitted by the laser system would traverse means for illuminating 120 in their center. Other shapes for means for illuminating 120 are possible. For example, means for illuminating may comprise one or more illuminants arranged randomly. One or more illuminants may be arranged in different portions of means for isolating 200, e.g., on extensible portion 220 and/or on intermediate portion 240 and/or on eye piece 260.

Means for illuminating 120 may emit visible light, infrared light or electromagnetic radiation of any other frequency and/or frequency spectrum. Visible light may help medical personnel in preparing, performing and/or supervising the corresponding treatment. Infrared light or other non-visible electromagnetic radiation may help sensors used for targeting of the laser and/or adjustment of the laser system and/or the apparatus 100 with respect to the patient and the position of his or her eye to undergo treatment. Such sensors may comprise eye trackers and/or proximity sensors.

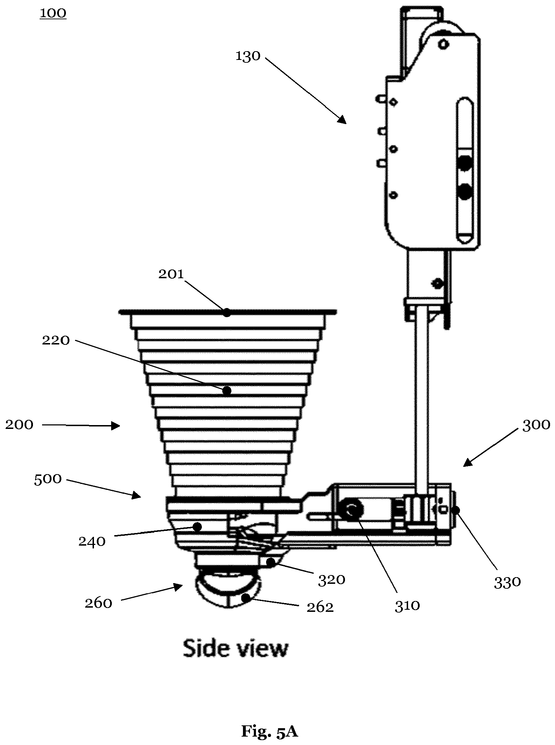

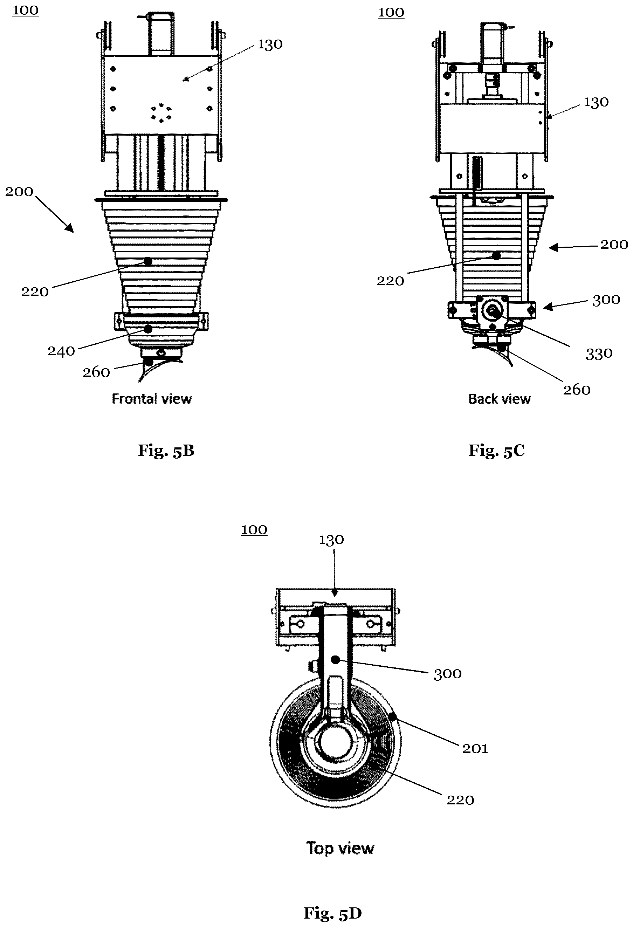

Figs. 5A-5D show an example of an apparatus 100 according to the present invention that comprises, in addition to what has been described above with reference to Figs. 1A-4D and the corresponding reference signs, means for extending 130 an extensible portion of means for isolating 200 such as extensible portion 220. In the example, means for extending 130 are arranged outside of the region circumscribed by means for isolating 200. Moreover, means for extending 130 are attached to unit 500. That is, extensible portion 220 is extended or collapsed indirectly as unit 500 and hence intermediate portion 240 is moved along the optical axis of the laser system by means for extending 130. In other examples, means for extending 130 may be arranged within or close to the region circumscribed by means for isolating 200. Additionally or alternatively, they may directly move an end such as rim 222 of an extensible portion such as extensible portion 220. In some examples, means for extending 130 may be controlled remotely, irrespective of the precise nature of the attachment of means for extending 130 to any portion of means for isolating 200 and/or unit 500. For example, means for extending 130 may comprise an electric engine that may be controlled via a remote control, e.g., by medical personnel, and/or they may be adapted for manual operation, e.g., by a lever. Means for extending 130 may be adapted to be firmly attached to the laser system, such as to provide safe anchoring of apparatus 100. In some examples, apparatus 100 may be provided with an electronic connection to the laser system via means for extending 130.

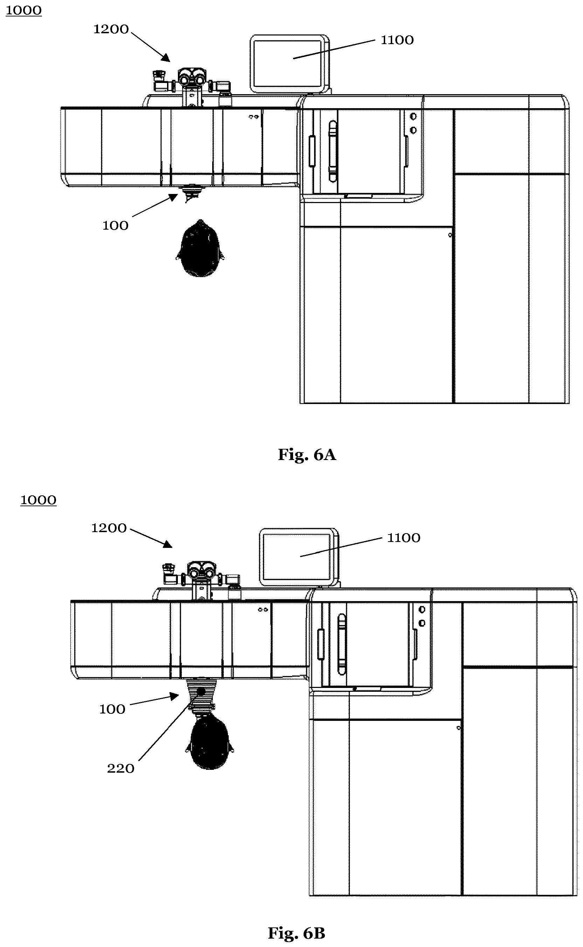

Means for extending 130 may be adapted to move apparatus 100 from at least one extended to at least one retracted position and vice versa. This may help a patient to more easily access the laser system for corneal tissue ablation as will be explained with reference to Figs. 6A, 6B.

Figs. 6A, 6B show a laser system 1000 that comprises apparatus 100 as well as a display 1100 and further controls 1200. Display 1100 may display information about laser system 1000 and/or apparatus 100, e.g., its position and/or degree of extension. Controls 1200 (and/or display 1100, e.g., if provided as a touchscreen) may be used to control the laser and/or apparatus 100, e.g., means for extending 130, means for illuminating 120, etc. Fig. 6A shows apparatus 100 in retracted position, for example while the patient accesses or exits laser system 1000, i.e., before the treatment begins or after it ends. Fig. 6B shows apparatus 100 in extended position, for example during the treatment, i.e., while the laser system is engaged.

## Claims

1. Apparatus (100) for providing a controlled atmosphere in a region between a laser system for corneal tissue ablation and an eye of a patient, comprising:
a. means for isolating (200) the region between the laser system and the eye, and
b. means for controlling (300) the atmosphere in the region,
c. wherein the means for isolating (200) comprise an eye piece (260) adapted to be arranged around the eye of the patient, and
d. wherein the eye piece (260) comprises a sleeve (262) adapted to provide a fit with the face.

2. Apparatus (100) according to claim 1, wherein the means for isolating (200) circumscribe the region between the laser system and the eye at least partly.

3. Apparatus (100) according to any of the preceding claims, wherein at least a portion (220) of the means for isolating (200) the region between the laser system and the eye is extensible.

4. Apparatus (100) according to claim 3, further comprising means for extending (130) the extensible portion (220) along an optical axis of the laser system.

5. Apparatus (100) according to any of the preceding claims, wherein the eye piece (260) is removable.

6. Apparatus (100) according to any of the preceding claims, wherein the means for isolating (200) are adapted to be arranged around a laser outlet of the laser system in a flush manner.

7. Apparatus (100) according to any of the preceding claims, wherein the means for controlling (300) the atmosphere are adapted to ventilate at least a portion of the region between the laser system and the eye by providing an airflow through the region, such that plumes are guided away from the patient.

8. Apparatus (100) according to claim 7, wherein the airflow is provided from at least one air inlet (262a) to at least one air outlet (262b) of the means for isolating (200).

9. Apparatus (100) according to claim 8, wherein the at least one air inlet in the means for isolating is arranged closer to the eye undergoing treatment than the at least one air outlet in the means for isolating.

10. Apparatus (100) according to claim 9, referring back to claim 5 or 6, wherein the at least one air inlet (262a) and/or the at least one air outlet (262b) is provided in the eye piece (260).

11. Apparatus (100) according to any of claims 8-10, wherein the means for controlling (300) the atmosphere further comprise a vacuum pump (310) which is connected to the at least one air outlet (262b).

12. Apparatus (100) according to any of the claims 7-11, wherein the means for controlling (300) the atmosphere further comprise a filter.

13. Apparatus (100) according to any of the preceding claims, further comprising means for illuminating the eye (120).

14. Laser system (1000) for corneal tissue ablation comprising an apparatus (100) according to any of the preceding claims.

## Patentansprüche

1. Vorrichtung (100) zum Bereitstellen einer kontrollierten Atmosphäre in einer Region zwischen einem Lasersystem zur Hornhautgewebeablation und einem Auge eines Patienten, umfassend:
a. Mittel zum Isolieren (200) der Region zwischen dem Lasersystem und dem Auge, und
b. Mittel zum Steuern (300) der Atmosphäre in der Region,
c. wobei die Mittel zum Isolieren (200) ein Augenstück (260) umfassen, das angepasst ist, um um das Auge des Patienten angeordnet zu werden, und
d. wobei das Augenstück (260) eine Hülse (262) umfasst, die angepasst ist, um eine Passung mit dem Gesicht bereitzustellen.

2. Vorrichtung (100) nach Anspruch 1, wobei die Mittel zum Isolieren (200) die Region zwischen dem Lasersystem und dem Auge zumindest teilweise umschreiben.

3. Vorrichtung (100) nach einem der vorhergehenden Ansprüche, wobei mindestens ein Abschnitt (220) der Mittel zum Isolieren (200) der Region zwischen dem Lasersystem und dem Auge verlängerbar ist.

4. Vorrichtung (100) nach Anspruch 3, ferner umfassend Mittel zum Verlängern (130) des verlängerbaren Abschnitts (220) entlang einer optischen Achse des Lasersystems.

5. Vorrichtung (100) nach einem der vorhergehenden Ansprüche, wobei das Augenstück (260) entfernbar ist.

6. Vorrichtung (100) nach einem der vorhergehenden Ansprüche, wobei die Mittel zum Isolieren (200) angepasst sind, um um einen Laserauslass des Lasersystems in einer bündigen Weise angeordnet zu werden.

7. Vorrichtung (100) nach einem der vorhergehenden Ansprüche, wobei die Mittel zum Steuern (300) der Atmosphäre angepasst sind, um mindestens einen Abschnitt der Region zwischen dem Lasersystem und dem Auge zu belüften, indem ein Luftstrom durch die Region bereitgestellt wird, sodass Dämpfe vom Patienten weggeführt werden.

8. Vorrichtung (100) nach Anspruch 7, wobei der Luftstrom von mindestens einem Lufteinlass (262a) zu mindestens einem Luftauslass (262b) der Mittel zum Isolieren (200) bereitgestellt wird.

9. Vorrichtung (100) nach Anspruch 8, wobei der mindestens eine Lufteinlass in den Mitteln zum Isolieren näher am Auge angeordnet ist, das einer Behandlung unterzogen wird, als der mindestens eine Luftauslass in den Mitteln zum Isolieren.

10. Vorrichtung (100) nach Anspruch 9, wenn auf Anspruch 5 oder 6 rückbezogen, wobei der mindestens eine Lufteinlass (262a) und/oder der mindestens eine Luftauslass (262b) im Augenstück (260) bereitgestellt ist.

11. Vorrichtung (100) nach einem der Ansprüche 8-10, wobei die Mittel zum Steuern (300) der Atmosphäre ferner eine Vakuumpumpe (310) umfassen, die mit dem mindestens einen Luftauslass (262b) verbunden ist.

12. Vorrichtung (100) nach einem der Ansprüche 7-11, wobei die Mittel zum Steuern (300) der Atmosphäre ferner einen Filter umfassen.

13. Vorrichtung (100) nach einem der vorhergehenden Ansprüche, ferner umfassend Mittel zum Beleuchten des Auges (120).

14. Lasersystem (1000) zur Hornhautgewebeablation, umfassend eine Vorrichtung (100) nach einem der vorhergehenden Ansprüche.

## Revendications

1. Appareil (100) pour produire une atmosphère contrôlée dans une région s'étendant entre un œil d'un patient et un système laser pour l'ablation du tissu de la cornée, comprenant :
a. des moyens d'isolation (200) de la région située entre l'œil et le système laser, et
b. des moyens de contrôle (300) de l'atmosphère dans la région,
c. dans lequel les moyens d'isolation (200) comprennent un œilleton (260) apte à être disposé autour de l'œil du patient, et
d. dans lequel l'œilleton (260) comprend un manchon (262) apte à permettre un ajustement avec le visage.

2. Appareil (100) selon la revendication 1, dans lequel les moyens d'isolation (200) font au moins partiellement le tour de la région s'étendant entre le système laser et l'œil.

3. Appareil (100) selon l'une des revendications précédentes, dans lequel au moins une partie (200) des moyens d'isolation (200) de la région s'étendant entre le système laser et l'œil est déployable.

4. Appareil (100) selon la revendication 3, comprenant en outre des moyens pour déployer (130) la partie déployable (220) suivant un axe optique du système laser.

5. Appareil (100) selon l'une des revendications précédentes, dans lequel l'œilleton (260) est amovible.

6. Appareil (100) selon l'une des revendications précédentes, dans lequel les moyens d'isolation (200) sont aptes à être agencés autour d'une sortie de laser du système laser au ras de celle-ci.

7. Appareil (100) selon l'une des revendications précédentes, dans lequel les moyens de contrôle (300) de l'atmosphère sont aptes à ventiler au moins une partie de la région s'étendant entre le système laser et l'œil en produisant un flux d'air traversant la région, de manière à guider vers l'extérieur du patient les particules.

8. Appareil (100) selon la revendication 7, dans lequel le flux d'air est délivré depuis au moins une entrée d'air (262a) vers au moins une sortie d'air (262b) des moyens d'isolation (200).

9. Appareil (100) selon la revendication 8, dans lequel l'au moins une entrée d'air des moyens d'isolation est agencée plus proche de l'œil faisant l'objet du traitement que l'au moins une sortie d'air des moyens d'isolation.

10. Appareil (100) selon la revendication 9, prise en dépendance de la revendication 5 ou 6, dans lequel l'au moins une entrée d'air (262a) et/ou l'au moins une sortie d'air (262b) sont situées dans l'œilleton (260).

11. Appareil (100) selon l'une des revendications 8 à 10, dans lequel les moyens de contrôle (300) de l'atmosphère comprennent en outre une pompe aspirante (310) qui est reliée à l'au moins une sortie d'air (262b) .

12. Appareil (100) selon l'une des revendications 7 à 11, dans lequel les moyens de contrôle (300) de l'atmosphère comprennent en outre un filtre.

13. Appareil (100) selon l'une des revendications précédentes, comprenant en outre des moyens d'illumination de l'œil (120).

14. Système laser (1000) pour l'ablation du tissu de la cornée, comprenant un appareil (100) selon l'une des revendications précédentes.
